# EUROPÄISCHE PATENTSCHRIFT

(11) **EP 1 765 766 B1**
(45) Veröffentlichungstag und Bekanntmachung des Hinweises auf die Patenterteilung: **25.09.2013**
(21) Anmeldenummer: 05757977.3
(22) Anmeldetag: 05.07.2005
(51) Int. Cl.: C07C 213/00, C07C 215/30

(54) **VERFAHREN ZUR HERSTELLUNG VON ENANTIOMEREN 3-HYDROXY-3- PHENYL-PROPYLAMINEN**
METHOD FOR PRODUCING ENANTIOMERS 3-HYDROXY-3-PHENYL-PROPYLAMINES
PROCEDE DE PRODUCTION DE 3-HYDROXY-3-PHENYLE-PROPYLAMINES ENANTIOMERES

(30) Priorität: 08.07.2004 DE 102004033313
(43) Veröffentlichungstag der Anmeldung: 28.03.2007
(73) Patentinhaber: Boehringer Ingelheim Pharma GmbH & Co. KG, 55216 Ingelheim (DE)
(72) Erfinder: BAUMGARTEN, Wolfgang, 55435 GAU-ALGESHEIM (DE); SCHIFFERS, Robert, 55435 GAU-ALGESHEIM (DE)
(74) Vertreter: Simon, Elke Anna Maria
(86) Internationale Anmeldenummer: PCT/EP2005/007207
(87) Internationale Veröffentlichungsnummer: WO 2006/005476

(56) Entgegenhaltungen:
- EP-A- 0 251 164
- DE-B3- 10 249 576
- US-B1- 6 187 956
- US-B1- 6 218 575
- TAKAHASHI H ET AL: "HIGHLY EFFICIENT ASYMMETRIC HYDROGENATION OF AMINO KETONE DERIVATIVES LEADING TO PRACTICAL SYNTHESES OF (S)-PROPRANOLOL AND RELATED COMPOUNDS" JOURNAL OF THE AMERICAN CHEMICAL SOCIETY, AMERICAN CHEMICAL SOCIETY, WASHINGTON, DC, US, Bd. 112, Nr. 15, 1990, Seiten 5876-5878, XP002914620 ISSN: 0002-7863
- S. SAKURABA: "Practical Asymmetric Synthesis of (R)-Fluoxetine Hydrochloride Catalyzed by (2S,4S)-4-Dicyclohexylphosphino-2-diphenyl phosphinomethyl-1-(N-methylcarbamoyl)pyrro lidine-Rhodium Complex" SYNLET, Bd. 10, 1991, Seiten 689-690, XP002353757
- S. SAKURABA: "Efficient Asymmetric Hydrogenation of gamma-Amino Ketone Hydrochloride Derivatives Ctalyzed by (2S,4S)-4-Dicyclohexylphosphino-2-diphenyl phosphinomethyl-1-(N-methylcarbamoyl)-pyrr olidine(MCCPM)-Rhodium Complex" SYNLETT, Bd. 10, 1992, Seiten 829-830, XP002353758

## Beschreibung

Die vorliegende Erfindung betrifft ein verbessertes Verfahren zur Herstellung von enantiomerenreinen 3-Hydroxy-3-Phenyl-propylaminen mittels Rhodium-katalysierter asymmetrischer Hydrierung im industriellen Maßstab.

### HINTERGRUND DER ERFINDUNG

Die reinen Enantiomere des 3-Hydroxy-3-phenyl-propylamin der Formel **I** 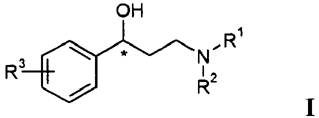 sind wertvolle Zwischenprodukte zur Synthese pharmazeutischer Wirkstoffe wie z.B. R-Atomoxetin, S-Fluoxetin oder S-Norfluoxetin, welche zu den pharmazeutisch als Antidepressivum oder Mittel zur Behandlung der Harninkontinenz eingesetzten Norepinephrine- und Serotonin-Aufnahme Inhibitoren gehören und von hohem kommerziellem Interesse sind. Die chemische Struktur der drei beispielhaft genannten Verbindungen sind in den Formeln **A-C** dargestellt:

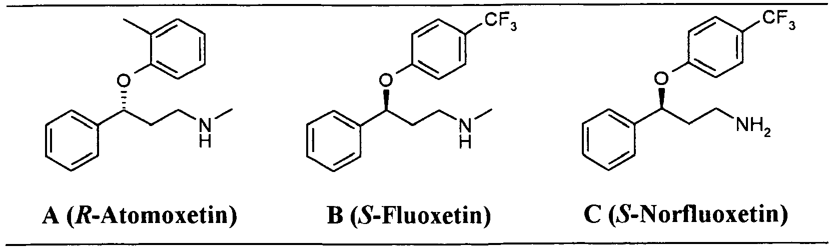

### STAND DER TECHNIK

Im Stand der Technik werden verschiedene Varianten zur Darstellung enantiomerenreiner Phenylpropylamine offenbart
- Sharpless et al. schlägt eine enantioselektive Epoxidierung vor (J. Org. Chem. 1988, 53, 4081) zur Darstellung von enantiomerenreinen Verbindungen dieser Klasse vor.
- Corey et al. schlägt eine enantioselektive Oxyborolidin-katalysierte Ketonreduktion (Tetrahedron Lett. 1989, 30 5207) zur Darstellung von enantiomerenreinen Verbindungen dieser Klasse vor.
- Koenig et al. zeigt eine klassische chemische Methode zur Herstellung eines racemischen Phenylpropylamins mit anschließender Racematspaltung durch chirale Mandelsäure (Tetrahedron Lett. 1994, 35, 1339).
- T. Ohkuma et al. schlägt in Organic Letters 2000, Vol. 2 No. 12 1749-1751 die enantioselektive Hydrierung von 3-Dimethylamino-1-(2-thienyl)-propanon mit Hilfe eines chiralen Ruthenium Katalysators in Gegenwart von Kalium *tert*-Butanolat vor.
- Takahashi et al. J of the American chemical Society, 112, 15, 1990, 5876-5878; Sakuraba et al. Synlett, 10, 1991, 689-690; Sakuraba et al. Synlet, 10, 1991, 829-830; US6187956B1; DE10249576B3 und US6218575B1 offenbaren die asymmetrische Hydrogenierung von Keto-Aminen zu den entsprechenden Hydroxy-Aminen. Als Katalysator wird in den Dokumenten chirales 4-(Dicyclohexylphosphino)-2-(diphenylphosphino-methyl)-N-methyl-aminocarbonylpyrrolidin eingesetzt.

Zur Herstellung von N-Benzyl-N-methyl-3-hydroxy-3-(2-thienyl)-propylaminen in industriellem Maßstab sind jedoch die im Stand der Technik beschriebenen Verfahren weniger geeignet, da entweder ungenügende optische Reinheiten erhalten werden, oder aber bei der enantioselektiven Reduktion große Mengen der schwerzugänglichen, teilweise instabilen chiralen Reduktionssysteme eingesetzt werden müssen.

Eines der wesentlichen Ziele der vorliegenden Erfindung ist es, ein Verfahren bereit zu stellen, durch das 3-Hydroxy-3-phenyl-propylamine I in hoher optischer und chemischer Reinheit, bevorzugt im technischen Maßstab, hergestellt werden können. Damit soll z.B. die Gefahr einer Verunreinigung eines Arzneistoffes mit den unerwünschten Enantiomeren minimiert werden.

Ein weiteres Ziel der Erfindung besteht darin, ein Verfahren bereit zu stellen, durch das weitgehend enantiomerenreine 3-Hydroxy-3-phenyl-propylamine ausgehend von leicht zugänglichen Ausgangsmaterialien in einfacher Weise dargestellt werden können.

Ein zusätzliches Ziel der Erfindung besteht darin, ein Verfahren bereit zu stellen, durch das *R*-Atomoxetin, S-Fluoxetin und S-Norfluoxetin in hoher optischer und chemischer Reinheit hergestellt werden können.

Überraschenderweise wurde nun gefunden, dass man 3-Hydroxy-3-phenyl-propylamine **I** als R- oder S-Enantiomer im technischen Maßstab in guten Ausbeuten und sehr guter optischer Reinheit erhalten kann, wenn man ein entsprechendes 1-Amino-3-(phenyl)-propan-3-on **II** 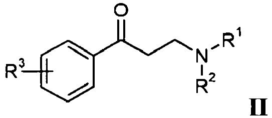 in einer asymmetrischen Hydrierung in Gegenwart von Rhodium und einem chiralen, zweizähnigen Phosphinliganden als Katalysatorsystem umsetzt.

### BESCHREIBUNG DER ERFINDUNG

Die vorliegende Erfindung betrifft ein Verfahren zur Herstellung von chiralen, optisch reinen 3-Hydroxy-3-phenyl-propylaminen, der Formel **I**, 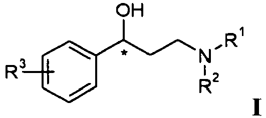 worin
- R¹: H, -C₁₋₆-alkyl oder -C₇₋₁₈-Aralkyl, bevorzugt H, Methyl, Ethyl, *iso*-Propyl, *tert-*Butyl oder Benzyl, besonders bevorzugt H, Methyl, oder Benzyl;
- R²: H, -C₁₋₆-alkyl oder -C₇₋₁₈-Aralkyl, bevorzugt H, Methyl, Ethyl, *iso*-Propyl, *tert-*Butyl oder Benzyl, besonders bevorzugt H, Methyl oder Benzyl;
- R³: H, -C₁₋₆-alkyl, -OH, -O-C₁₋₆-alkyl, -O-C₇₋₁₈-Aralk-yl, -OOC-C₁₋₆-Alkyl, -OOC-Aryl, Halogen, bevorzugt, H, Methyl, *iso*-Propyl, *tert*-Butyl, Hydroxy, Methoxy, Propoxy, Butoxy, Benzyloxy, Acetyloxy, Benzoyloxy, F, Cl oder Br; besonders bevorzugt H, Hydroxy, Methoxy, Benzyloxy, Acetyloxy oder Benzoyloxy;
bedeutet, oder ein Säureadditionssalz davon, ausgehend von 1-Amino-3-(phenyl)-propan-3-on der Formel **II**, 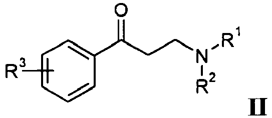 worin R¹, R², und R³ die oben genannte Bedeutung haben, oder einem Säureadditionssalz davon, dadurch gekennzeichnet, dass man dieses (II) einer asymmetrischen Hydrierung in Gegenwart eines Katalysatorsystems bestehend aus Rhodium und chiralem 4-(Dicyclohexylphosphino)-2-(diphenylphosphino-methyl)-N-methyl-aminocarbonyl-pyrrolidin, in Gegenwart von weniger als einem Äquivalent einer schwachen Base ausgewählt aus der Gruppe der Alkalimetallhydrogencarbonate, Alkalimetallcarbonate und in einem inerten, protischen Verdünnungsmittels, das Wasser enthält unterwirft und zur Isolierung des Produktes folgende Schritte durchgeführt werden:
(i) Verteilung der bei der asymmetrischen Hydrierung erhaltenen Reaktionsmischung zwischen Wasser und einem organischen Lösungsmittel,
(ii) Einstellen eines pH-Wertes der wässrigen Phase im sauren Bereich,
(iii) Abtrennen der wässrigen Phase,
(iv) gegebenenfalls Wiederholung der Schritte (i) bis (iii)
(v) Einstellen des pH-Wertes der wässrigen Phase im basischen Bereich;
(vi) Verteilung der Reaktionsmischung zwischen Wasser und einem organischen Lösungsmittel,
(vii) gegebenenfalls Wiederholung der Schritte (v) bis (vi)
(vii) Abtrennen der gebildeten organischen Phase und Konzentrierung.

Das als Ausgangsprodukt einzusetzende **II** erhält man durch Umsetzung des mit R³-substituierten 1-Phenylethanons mit einem entsprechend substituierten Amin und Formaldehyd in einer Mannich-Reaktion. Bevorzugt erfolgt die enantioselektive Hydrierung in Abwesenheit eines Diamins.

Bevorzugt ist das obige Verfahren zur Herstellung von chiralen 3-Hydroxy-3-phenyl-propylaminen, der Formel I, worin
- R¹: H, Methyl, Ethyl, *iso*-Propyl, *tert*-Butyl oder Benzyl;
- R²: H, Methyl, Ethyl, *iso*-Propyl, *tert*-Butyl oder Benzyl;
- R³: H, Methyl, iso-Propyl, *tert*-Butyl, Hydroxy, Methoxy, Propoxy, Butoxy, Benzyloxy, Acetyloxy, Benzoyloxy, F, Cl oder Br;
bedeutet.

Bevorzugt ist das obige Verfahren zur Herstellung von chiralen 3-Hydroxy-3-phenyl-propylaminen, der Formel **I**, worin
- R¹: H, Methyl oder Benzyl;
- R²: H, Methyl oder Benzyl;
- R³: H, Hydroxy, Methoxy, Benzyloxy, Acetyloxy oder Benzoyloxy;
bedeutet.

Bevorzugt ist das obige Verfahren zur Herstellung von chiralen 3-Hydroxy-3-phenyl-propylaminen, der Formel I, worin
- R¹: H, Methyl oder Benzyl;
- R²: H, Methyl oder Benzyl;
- R³: H;
bedeutet.

Ein Katalysatorsystem bestehend aus Rhodium und (2*R*, 4*R*)-4-(Dicyclohexylphosphino)-2-(diphenylphosphino-methyl)-N-methyl-aminocarbonyl-pyrrolidin (*RR*-MCCPM) führt zum entsprechenden *S*-3-Hydroxy-3-phenyl-propylamin **I**-***S*** als Produkt.

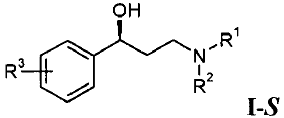

Bevorzugt ist deshalb das obige Verfahren zur Herstellung von *S*-3-Hydroxy-3-phenyl-propylaminen **I**-***S***, worin das Katalysatorsystem aus Rhodium und (2*R*, 4*R*)-4-(Dicyclohexylphosphino)-2-(diphenylphosphino-methyl)-N-methyl-aminocarbonyl-pyrrolidin besteht.

Ein Katalysatorsystem bestehend aus Rhodium und (2*S*, 4*S*)-4-(Dicyclohexylphosphino)-2-(diphenylphosphino-methyl)-N-methyl-aminocarbonyl-pyrrolidin (*SS-*MCCPM) führt zum entsprechenden *R*-3-Hydroxy-3-phenyl-propylamin **I**-***R*** als Produkt.

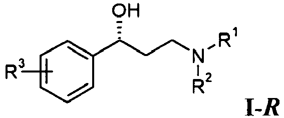

Bevorzugt ist deshalb das obige Verfahren zur Herstellung von *R*-3-Hydroxy-3-phenylpropylaminen **I**-***R***, worin das Katalysatorsystem aus Rhodium und (2*S*, 4*S*)-4-(Dicyclohexylphosphino)-2-(diphenylphosphino-methyl)-N-methyl-aminocarbonyl-pyrrolidin besteht.

Die Reaktion wird gemäß Ansprunch 1 in Gegenwart einer schwachen Base durchgeführt. Bevorzugt werden Na₂CO₃, K₂CO₃, oder NaHCO₃ verwendet.

Bevorzugt ist ein Verfahren, wobei die asymmetrische Hydrierung in einem Temperaturbereich von 0°C bis 100°C, vorzugsweise von 0°C bis 50°C, insbesondere von 20°C bis 40°C durchgeführt wird.

Weiterhin bevorzugt ist ein Verfahren, wobei die asymmetrische Hydrierung unter einem Druck von 1-150 bar, vorzugsweise unter einem Druck von 50-150 bar, insbesondere bei etwa 80-120 bar, besonders bevorzugt 100 bar durchgeführt wird.

Als inerte Verdünnungsmittel werden protische Lösungsmittel, bevorzugt verzweigte oder unverzweigte C₁₋₈-Alkohole, die Wasser enthalten eingesetzt. Besonders bevorzugt werden niedere Alkohole wie Methanol, Ethanol, *n*-Propanol und *iso-*Propanol oder deren Mischungen eingesetzt. Besonders bevorzugt wird als Reaktionsmedium Methanol verwendet, wobei das Methanol oder die anderen Alkohole oder Lösungsmittel Wasser enthält.

Bevorzugt sind solche Verfahren, wobei man **II** oder dessen Säureadditionssalze zum Rhodiumkatalysator bei der asymmetrischen Hydrierung in einem Mol-Verhältnis von 500:1 bis 100000:1, vorzugsweise von 750:1 bis 20000:1 einsetzt.

Bei einem Mol-Verhältnis Katalysator zu Substrat von etwa 1:2000 erhält man durch das erfindungsgemäße Verfahren ausgehend von 1-(N-Alkyl-N-methylamino)-3-(2-thienyl)-propan-3-on Hydrochlorid (S)-N-Alkyl-N-methyl-3-hydroxy-3-(2-thienyl)-propylamine bereits in einer optischen Reinheit von > 94 % ee.

Die Herstellung dieses Katalysators ist aus dem Stand der Technik bekannt [EP 0 251 164 und EP 0 336 123]. Der Katalysator kann auch polymergebunden vorliegen, z.B. indem der chirale Ligand 4-Dicyclohexylphosphino)-2-(diphenylphosphino-methyl)-N-methyl-aminocarbonyl)pyrrolidin z.B. über die Phenylgruppen an ein Polymer gebunden ist. Dabei schließt die Verwendung solcher polymergebundener Liganden den gleichzeitigen Einsatz von nicht-polymergebundenem Liganden nicht zwingend aus. Solche polymergebunden Katalysatoren sind insbesondere für eine einfache Reinigung des Produktes von Vorteil.

Der Katalysator wird entweder als vorgefertigte, sauerstofffreie Lösung von [Rh(COD)Cl₂]₂ und Ligand eingesetzt oder in situ aus [Rh(COD)Cl₂]₂ und Ligand in Gegenwart von 1-Amino-3-phenyl-propan-3-on sauerstofffrei unter Schutzgasatmosphäre oder Wasserstoffatmosphäre hergestellt.

Zur Darstellung eines *S*-Enantiomers der Formel **I**-***S*** wird als Katalysator erfindungsgemäß [Rh(COD)Cl₂]₂, wobei COD für eine Cyclooctadienylgruppe steht, und (2*R*, 4*R*)-4-(Dicyclohexylphosphino)-2-(diphenylphosphino-methyl)-N-methyl-aminocarbonylpyrrolidin (*RR-*MCCPM) als chiraler, zweizähniger Phosphinligand (PP*) eingesetzt.

Zur Darstellung eines *R*-Enantiomers der Formel **I**-***R*** wird als Katalysator erfindungsgemäß [Rh(COD)Cl₂]₂, wobei COD für eine Cyclooctadienylgruppe steht, und (2*S*, 4*S*)-4-(Dicyclohexylphosphino)-2-(diphenylphosphino-methyl)-N-methyl-aminocarbonylpyrrolidin (*SS*-MCCPM) als chiraler, zweizähniger Phosphinligand (PP*) eingesetzt.

Die Hydrierung wird in der Regel sauerstofffrei, zweckmäßigerweise unter Inertgas, durchgeführt, bevorzugt unter Wasserstoffatmosphäre. Für die Reaktion ist es jedoch nicht zwingend, dass der Wasserstoff für die Hydrierung aus dem Atmosphärengas über der Reaktionsmischung entnommen werden kann. Der Wasserstoff kann auch in situ in Lösung aus geeigneten Wasserstoffquellen erzeugt werden. Zu solchen Wasserstoffquellen zählen z.B. Ammoniumformiat, Ameisensäure und andere Formiate, Hydrazine in Gegenwart von Metallionen wie Fe²⁺/Fe³⁺ und andere aus dem Stand der Technik bekannte Wasserstoffquellen.

Die Reaktionszeit für die asymmetrische Hydrierung beträgt in der Regel bis zu ihrer Beendigung zwischen 2 und 48 Stunden, bevorzugt liegt sie zwischen 4 und 36 Stunden, besonders bevorzugt beträgt sie etwa 18 bis 22 Stunden.

Die Aufarbeitung der Reaktion erfolgt gemäß Anspruch 1.

Das Einstellen des pH-Wertes der wässrigen Phase im sauren Bereich in Schritt (ii) dient dazu, das Salz es hydrierten Produktes zu bilden, so dass die Löslichkeit des Produktes in der wässrigen Phase erhöht wird. Der dazu eingestellte pH-Wert hängt vom Produkt ab, bevorzugt wird ein pH-Wert von 1-2, besonders bevorzugt 1,2-1,8. Das Einstellen des pH-Wertes der wässrigen Phase im basischen Bereich in Schritt (v) dazu, das hydrierte Produktes aus seiner Salzform heraus zu bringen, so dass die Löslichkeit des Produktes in der organischen Phase erhöht wird. Der dazu eingestellte pH-Wert hängt wiederum vom Produkt ab, bevorzugt wird ein pH-Wert von 6-10, besonders bevorzugt 7-9.

Insbesondere wird zur Aufarbeitung und Isolierung des Produktes nach der enantioselektiven Hydrierung die erhaltene Reaktionsmischung eingeengt und der erhaltene Feststoff zwischen Wasser und einem organischem Lösungsmittel, bevorzugt Toluol oder Dichlormethan verteilt. Der pH-Wert der wässrigen Phase wird auf einen Wert von 1 bis 2, vorzugsweise 1,2 bis 1,8, eingestellt und anschließend die Wasserphase abgetrennt. Die organische Phase wird vorzugsweise nochmals mit Wasser versetzt, angesäuert, und erneut abgetrennt. Die vereinigten Wasserphasen werden auf einen pH von 8 bis 10, vorzugsweise 8,5 bis 9,5, eingestellt, mit Lösungsmittel versetzt und extrahiert. Das entsprechende 3-Hydroxy-3-phenyl-propylamin der Formel I wird nach Entfernung des Lösungsmittels mit hoher chemischer (im allgemeinen > 96%) und optischer Reinheit (in der Regel > 94% ee) erhalten.

In einer bevorzugten Ausführungsform des Verfahrens wird das Produkt der katalytischen Hydrierung anschließend zu einem Salz umgewandelt. Dies dient dazu, die Isolierung der jeweiligen 3-Hydroxy-3-phenyl-propylamine zu erleichtern und die Enantiomerenreinheit weiter zu steigern, bevorzugt auf Werte > 99%. Weiterhin werden so transportier- und lagerbare Feststoffe erhalten. So können z.B. Salze mit anorganischen Säuren wie Salzsäure, Schwefelsäure, Bromwasserstoffsäure, Phosphorsäure, Sulfonsäure oder organischen Säuren, wie Oxalsäure, Maleinsäure, Fumarsäure, Zitronensäure, Bernsteinsäure oder Essigsäure, gebildet werden. Ferner können Mischungen der vorgenannten Säuren eingesetzt werden. Zur Erhöhung der Enantiomerenreinheit können, aber müssen nicht notwendigerweise, ebenfalls chirale Salzbildner eingesetzt werden wie zum Beispiel chirale Mandelsäure, Milchsäure oder Weinsäure.

Besonders bevorzugt wird das obige Verfahren zur Herstellung von Verbindung der Formel **I-A**, 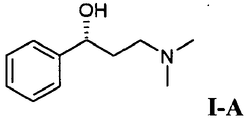 die in weiteren Reaktionsschritten zu R-Atomoxetin umgesetzt wird. Ebenfalls besonders bevorzugt wird das obige Verfahren zur Herstellung von Verbindung der Formel **I-B**, 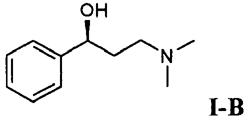 die in weiteren Reaktionsschritten ebenfalls zu R-Atomoxetin oder aber zu S-Fluoxetin umgesetzt wird. Ebenfalls besonders bevorzugt wird das obige Verfahren zur Herstellung einer Verbindung der Formel I-C, 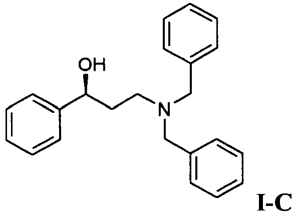 die in weiteren Reaktionsschritten zu S-Norfluoxetin umgesetzt wird.

### VERWENDETE BEGRIFFE UND DEFINITIONEN

Unter dem Begriff "C₁₋₆-Alkyl" (auch soweit sie Bestandteil anderer Reste sind) werden verzweigte und unverzweigte Alkylgruppen mit 1 bis 6 Kohlenstoffatomen verstanden, dem entsprechend werden unter dem Begriff "C₁₋₄Alkyl" verzweigte und unverzweigte Alkylgruppen mit 1 bis 4 Kohlenstoffatomen verstanden. Bevorzugt sind Alkylgruppen mit 1 bis 4 Kohlenstoffatome. Beispielsweise werden hierfür genannt: Methyl, Ethyl, *n-*Propyl, *iso*-Propyl, *n*-Butyl, *iso*-Butyl, *sec*-Butyl, *tert*-Butyl, *n*-Pentyl, *iso*-Pentyl, *neo-*Pentyl oder Hexyl. Gegebenenfalls werden für vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Unter dem Begriff "C₁₋₈-Alkohol" werden verzweigte und unverzweigte Alkohole mit 1 bis 8 Kohlenstoffatomen verstanden und einer oder zwei Hydroxygruppen. Dem entsprechend werden unter dem Begriff "C₁₋₄-Alkohol" verzweigte und unverzweigte Alkylgruppen mit 1 bis 4 Kohlenstoffatomen und einer oder zwei Hydroxygruppen verstanden. Bevorzugt sind Alkohole mit 1 bis 4 Kohlenstoffatome. Beispielsweise werden hierfür genannt: Methanol, Ethanol, *n*-Propanol, iso-Propanol, *n*-Butanol, *iso-*Butanol, *sec*-Butanol, *tert*-Butanol, *n*-Pentanol, *iso*-Pentanol, *neo*-Pentanol oder Hexanol. Gegebenenfalls werden für vorstehend genannten Moleküle auch die Abkürzungen MeOH, EtOH, *n*-PrOH, *i*-PrOH, *n*-BuOH, *i*-BuOH, *t*-BuOH, etc. verwendet. Sofern nicht anders beschrieben, umfassen die Definitionen Propanol, Butanol, Pentanol und Hexanol alle denkbaren isomeren Formen der jeweiligen Reste. So umfasst beispielsweise Propanol *n*-Propanol und *iso*-Propanol, Butanol umfasst *iso-*Butanol, *sec*-Butanol und *tert*-Butanol etc.

Unter dem Begriff "Aryl" (auch soweit sie Bestandteil anderer Reste sind) werden aromatische Ringsysteme mit 6 oder 10 Kohlenstoffatomen verstanden. Beispielsweise werden hierfür genannt: Phenyl oder Naphthyl, bevorzugter Arylrest ist Phenyl. Soweit nicht anders beschrieben, können die Aromaten substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff " C₇₋₁₈-Aralkyl" (auch soweit sie Bestandteil anderer Reste sind) werden verzweigte und unverzweigte Alkylgruppen mit 1 bis 8 Kohlenstoffatomen verstanden, die mit einem aromatische Ringsysteme mit 6 oder 10 Kohlenstoffatomen substituiert sind, entsprechend werden unter dem Begriff "C₇₋₁₁-Aralkyl" verzweigte und unverzweigte Alkylgruppen mit 1 bis 4 Kohlenstoffatomen verstanden, die mit einem aromatische Ringsysteme mit 6 Kohlenstoffatomen substituiert sind. Beispielsweise werden hierfür genannt: Benzyl, 1- oder 2-Phenylethyl. Soweit nicht anders beschreiben, können die Aromaten substituiert sein mit einem oder mehreren Resten ausgewählt aus der Gruppe bestehend aus Methyl, Ethyl, *iso*-Propyl, *tert-Butyl,* Hydroxy, Fluor, Chlor, Brom und Jod.

Gegebenenfalls können die Verbindungen der Formel **I** und **II** in ihre Säureadditionssalze mit einer anorganischen oder organischen Säure, überführt werden. Als Säuren kommen hierfür beispielsweise Bernsteinsäure, Bromwasserstoffsäure, Essigsäure, Fumarsäure, Maleinsäure, Methansulfonsäure, Milchsäure, Oxalsäure, Phosphorsäure, Salzsäure, Schwefelsäure, Weinsäure oder Zitronensäure in Betracht. Ferner können Mischungen der vorgenannten Säuren eingesetzt werden.

Das erfindungsgemäße Verfahren soll nun durch die nachfolgenden Beispiele erläutert werden.

### BEISPIELE

### I) Synthese der Keton-Vorstufen

1) **3-N,N-Dimethylamino-1-phenyl-propan-1-on-Hydrochlorid:** 480,6 g ( 4,0 mol) Acetophenon werden in 1,0 L iso-Propanol gelöst und unter Rühren 132,1 g ( 4,4 mol) Paraformaldehyd zugesetzt, anschließend werden 326,2 g (4,0 mol) Dimethylamin-Hydrochlorid eingetragen und mit weiteren 100 ml *iso*-Propanol nachgespült. Es wird für fünf Stunden auf 90°C erhitzt, danach auf etwa 4°C abgekühlt und der entstandene Feststoff abgesaugt und mit 200 mL kaltem iso-Propanol und anschließend mit 600 mL Aceton gewaschen. Die 557,6 g Rohprodukt werden anschließend bei 50°C im Vakuum getrocknet, Ausbeute 512,4 g (59,9%), Reinheit >98% nach NMR.
2) **3-(Benzyl-methyl-amino)-1-phenyl-propan-1-on-Hydrochlorid:** 114,0 g ( 0,95 mol) Acetophenon werden in 73 mL Ethanol gelöst und unter Rühren 28,5 g ( 0,95 mol) Paraformaldehyd zugesetzt, anschließend werden 149,8 g (0,95 mol) N-Benzyl, N-methylamin-Hydrochlorid eingetragen und mit weiteren 30 ml Ethanol nachgespült. Es wird für fünf Stunden auf 82°C erhitzt, danach auf etwa 20°C abgekühlt, mit 50 mL Ethanol verdünnt und der entstandene Feststoff abgesaugt und mit 150 mL Ethanol gewaschen. Die 237 g Rohprodukt werden anschließend bei 50°C im Vakuum getrocknet, Ausbeute 212,8 g (77,3%), Reinheit > 97% nach HPLC.

### II) Hydrierungen

1) **(S)-3-N,N-Dimethylamino-1-phenyl-propan-1-ol**: 373 g (1,745 mol) 3-N,N-Dimethylamino-1-phenyl-propan-1-on-Hydrochlorid werden in 1,65 Liter Methanol und 0,19 Liter Wasser unter Stickstoff suspendiert, 88 mg Bis-(1,5-cyclooctadien)-dirhodium(I)-dichlorid, 187 mg *RR*-MCCPM-Ligand und 746 mg Natriumhydrogencarbonat hinzugefügt und die Suspension bei 30°C und 100 bar Wasserstoffdruck für etwa 10 Stunden hydriert. Die Reaktionsmischung wird eingeengt und der erhaltene Rückstand zwischen 1,0 L Wasser und 0,4 L organischem Lösungsmittel (Toluol oder Dichlormethan) verteilt. Mit 32%iger Salzsäure wird ein pH-Wert von 1,5 eingestellt und für 10 Minuten gerührt, anschließend wird die Wasserphase abgetrennt. Die organische Phase wird nochmals mit 0,3 L Wasser versetzt, gerührt und die Wasserphase wiederum abgetrennt. Die vereinigten Wasserphasen werden nun mit 1,0 L organischem Lösungsmittel und 45%iger Natronlauge auf einen pH von 9,0 eingestellt, gerührt, und anschließend die Phasen getrennt. Die Wasserphase wird nochmals mit 0,5 L Lösungsmittel extrahiert und die vereinigten organischen Phasen bei 60°C und 7 mbar eingeengt. Die Ausbeute beträgt 271,7 g (86,9%), Reinheit >98% (NMR), Enantiomerenreinheit 94,5% (HPLC).
2) **(S)-3-N-Benzyl,N-methylamino-1-phenyl-propan-1-ol**: 15 g (52 mmol) 3-(Benzyl-methyl-amino)-1-phenyl-propan-1-on-Hydrochlorid werden in 135 mL Methanol und 15 mL Wasser unter Stickstoff suspendiert, 5,7 mg Bis-(1,5-cyclooctadien)-dirhodium(I)-dichlorid, 12,1 mg *RR-*MCCPM-Ligand und 30 mg Natriumhydrogencarbonat hinzugefügt und die Suspension bei 30°C und 100 bar Wasserstoffdruck für etwa 24 Stunden hydriert. Die Reaktionsmischung wird eingeengt und der erhaltene Rückstand zwischen 70 mL Wasser und 70 mL organischem Lösungsmittel (Toluol oder Dichlormethan) verteilt. Mit 32%iger Salzsäure wird ein pH-Wert von 1,4 eingestellt und für 10 Minuten gerührt, anschließend die Wasserphase abgetrennt. Die organische Phase wird nochmals mit 40 mL Wasser versetzt, gerührt und die Wasserphase wiederum abgetrennt. Die vereinigten Wasserphasen werden nun nochmals mit 10 mL organischem Lösungsmittel extrahiert, anschließend mit 60 mL Lösungsmittel versetzt und mit 45%iger Natronlauge auf einen pH von 6,4 eingestellt, gerührt, und anschließend die Phasen getrennt. Die Wasserphase wird nochmals mit 30 mL Lösungsmittel extrahiert und die vereinigten organischen Phasen bei 60°C und 5 mbar eingeengt. Die Ausbeute beträgt 10,72 g (80,8%), Reinheit >98% (NMR), Enantiomerenreinheit 94% (NMR).

### III) Salze der hydrierten Aminoalkohole

1) **(S)-3-N,N-Dimethylamino-1-phenyl-propan-1-ol-Oxalat**: 17,9 g (S)-3-N,N-Dimethylamino-1-phenyl-propan-1-ol, Enantiomerenreinheit 94% (NMR) wird in 188 ml *iso*-Propanol gelöst und anschließend 9,0g Oxalsäure unter Rühren zugesetzt. Die Mischung wird mit weiteren 107 ml iso-Propanol versetzt und für 15 Minuten zum Rückfluss erhitzt, anschließend auf etwa 40°C abgekühlt. Die entstandene dicke Suspension wird mit weiteren 36 ml *iso*-Propanol versetzt und auf etwa 20°C abgekühlt, anschließend filtriert und mit 100 ml *iso*-Propanol gewaschen. Das Rohprodukt wird bei 50°C im Vakuum getrocknet und es werden 24,0 g (89% d. Th.) an (S)-3-N,N-Dimethylamino-1-phenyl-propan-1-ol-Oxalat in Form weißer Kristalle, Enantiomerenreinheit **96,3%** (HPLC), Schmelzpunkt 125-126°C erhalten.
2a) **(S)-3-N-Benzyl,N-methylamino-1-phenyl-propan-1-ol-Mandelat**: 2,55 g (S)-3-N-Benzyl,N-methylamino-1-phenyl-propan-1-ol, Enantiomerenreinheit 94% (NMR) wird in 8,1 ml Toluol gelöst und 1,52 g D-(-)-Mandelsäure unter Rühren zugesetzt. Die Mischung wird mit 8,1 ml Toluol verdünnt, auf etwa 3°C abgekühlt und die Kristalle abgesaugt und mit 12 ml kaltem Toluol gewaschen. Nach Trocknung bei 50°C im Vakuum werden 3,37 g (83%) des (S)-3-N-Benzyl,N-methylamino-1-phenyl-propan-1-ol-Mandelates, Enantiomerenreinheit 100% (HPLC), Schmelzpunkt 142-143°C erhalten.
2b) **(S)-3-N-Benzyl,N-methylamino-1-phenyl-propan-1-ol-Hydrochlorid**: 2,55 g (S)-3-N-Benzyl,N-methylamino-1-phenyl-propan-1-ol, Enantiomerenreinheit 94% (NMR) wird in 5,8 ml Toluol gelöst und 1,83 g 20%ige isopropanolische Salzsäure unter Rühren zugesetzt. Die Mischung wird auf etwa 3°C abgekühlt, die Kristalle abgesaugt und mit 9,5 ml kaltem Toluol gewaschen. Nach Trocknung bei 60°C im Vakuum werden 1,69 g (58%) des (S)-3-N-Benzyl,N-methylamino-1-phenyl-propan-1-ol-Hydrochlorids, Enantiomerenreinheit 95,4% (HPLC) erhalten.

## Patentansprüche

1. Verfahren zur Herstellung von chiralem 3-Hydroxy-3-phenyl-propylaminen, der Formel I, 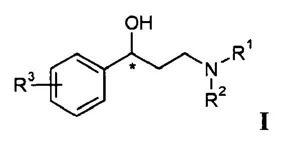 worin
R¹ H, -C₁₋₆-alkyl oder -C₇₋₁₈-Aralkyl;
R² H, -C₁₋₆-alkyl oder -C₇₋₁₈-Aralkyl;
R³ H, -C₁₋₆-alkyl, -OH, -O-C₁₋₆-alkyl, -O-C₇₋₁₈-Aralkyl, -OOC-C₁₋₆-Alkyl, -OOC-Aryl oder Halogen;
bedeutet, oder ein Säureadditionssalz davon, ausgehend von 1-Amino-3-(phenyl)-propan-3-on der Formel **II**, 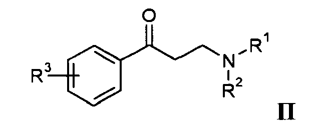 worin R¹, R², und R³ die oben genannte Bedeutung haben, oder einem Säureadditionssalz davon, **dadurch gekennzeichnet, dass** man dieses (II) einer asymmetrischen Hydrierung in Gegenwart eines Katalysatorsystems bestehend aus Rhodium und chiralem 4-(Dicyclohexylphosphino)-2-(diphenylphosphino-methyl)-N-methyl-aminocarbonyl-pyrrolidin, in Gegenwart von weniger als einem Äquivalent einer schwachen Base ausgewählt aus der Gruppe der Alkalimetallhydrogencarbonate, Alkalimetallcarbonate und in einem inerten, protischen Verdünnungsmittels, das Wasser enthält unterwirft und zur Isolierung des Produktes folgende Schritte durchgeführt werden:
(i) Verteilung der bei der asymmetrischen Hydrierung erhaltenen Reaktionsmischung zwischen Wasser und einem organischen Lösungsmittel,
(ii) Einstellen eines pH-Wertes der wässrigen Phase im sauren Bereich,
(iii) Abtrennen der wässrigen Phase,
(iv) gegebenenfalls Wiederholung der Schritte (i) bis (iii)
(v) Einstellen des pH-Wertes der wässrigen Phase im basischen Bereich;
(vi) Verteilung der Reaktionsmischung zwischen Wasser und einem organischen Lösungsmittel,
(vii) gegebenenfalls Wiederholung der Schritte (v) bis (vi)
(vii) Abtrennen der gebildeten organischen Phase und Konzentrierung.

2. Verfahren nach Anspruch 1 worin
R¹ H, Methyl, Ethyl, iso-Propyl, *tert-*Butyl oder Benzyl;
R² H, Methyl, Ethyl, *iso*-Propyl, tert-Butyl oder Benzyl;
R³ H, Methyl, *iso*-Propyl, *tert-*Butyl*,* Hydroxy, Methoxy, Propoxy, Butoxy, Benzyloxy, Acetyloxy, Benzoyloxy, F, Cl oder Br;
bedeutet.

3. Verfahren zur Herstellung von S-3-Hydroxy-3-phenyl-propylaminen **I**-***S*** nach einem der Ansprüche 1 oder 2, 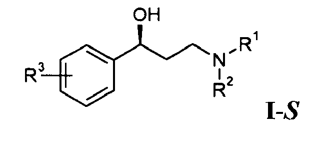 worin das Katalysatorsystem aus Rhodium und (2*R*, 4*R*)-4-(Dicyclo-hexylphosphino)-2-(diphenylphosphino-methyl)-N-methyl-aminocarbonyl-pyrrolidin besteht.

4. Verfahren zur Herstellung von *R*-3-Hydroxy-3-phenyl-propylaminen **I**-***R*** nach einem der Ansprüche 1 oder 2, 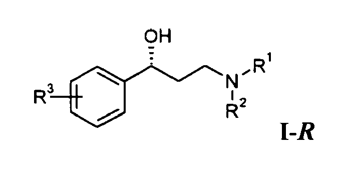 worin das Katalysatorsystem aus Rhodium und (2*S*, 4*S*)-4-(Dicyclo-hexylphosphino)-2-(diphenylphosphino-methyl)-N-methyl-aminocarbonyl-pyrrolidin besteht.

5. Verfahren nach einem der Ansprüche 1 bis 4, **dadurch gekennzeichnet, dass** die asymmetrische Hydrierung in einem Temperaturbereich von 0°C bis 100°C durchgeführt wird.

6. Verfahren nach einem der Ansprüche 1 bis 5, **dadurch gekennzeichnet, dass** die asymmetrische Hydrierung unter einem Druck von 1 bis 150 bar durchgeführt wird.

7. Verfahren nach einem der Ansprüche 1 bis 6, worin man eine Verbindung der Formel **II** oder ein Säureadditionssalz davon zum Rhodiumkatalysator bei der asymmetrischen Hydrierung in einem Mol-Verhältnis von 500:1 bis 100000:1 einsetzt.

8. Verfahren nach einem der Ansprüche 1 bis 7, worin der Rhodiumkatalysator für die asymmetrische Hydrierung als vorgefertigte Lösung eingesetzt wird.

9. Verfahren nach einem der Ansprüche 1 bis 7, worin der Rhodiumkatalysator für die asymmetrische Hydrierung in situ erzeugt wird.

10. Verfahren nach einem der Ansprüche 1-9, worin die asymmetrische Hydrierung innerhalb einer Reaktionszeit von 2 bis 48 Stunden durchgeführt wird.

11. Verfahren nach einem der vorangegangenen Ansprüche worin das Produkt der katalytischen Hydrierung anschließend zu einem Salz umgewandelt wird.

12. Verfahren zur Herstellung von *R*-Atomoxetin wobei zunächst die Verbindung **I-A** hergestellt wird gemäß dem Verfahren nach einem der Ansprüche 1 bis 11 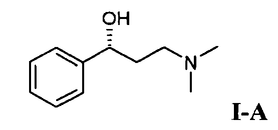 und anschließend in weiteren Reaktionsschritten zu *R*-Atomoxetin umgesetzt wird.

13. Verfahren zur Herstellung von *R*-Atomoxetin wobei zunächst die Verbindung **I-B** hergestellt wird gemäß dem Verfahren nach einem der Ansprüche 1 bis 11 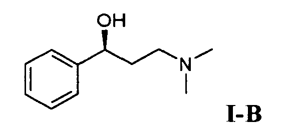 und anschließend in weiteren Reaktionsschritten zu *R*-Atomoxetin umgesetzt wird.

14. Verfahren zur Herstellung von *R*-Atomoxetin wobei zunächst die Verbindung **I-C** hergestellt wird gemäß dem Verfahren nach einem der Ansprüche 1 bis 11 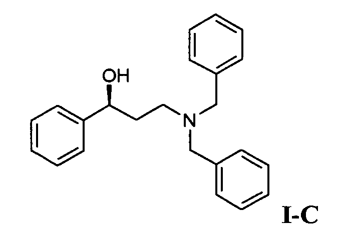 und anschließend in weiteren Reaktionsschritten zu *R*-Atomoxetin umgesetzt wird.

## Claims

1. Process for preparing chiral 3-hydroxy-3-phenyl-propylamines of formula I, 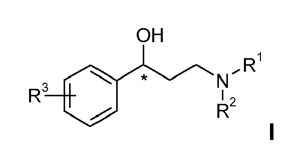 wherein
R¹ denotes H, -C₁₋₆-alkyl or -C₇₋₁₈-aralkyl;
R² denotes H, -C₁₋₆-alkyl or -C₇₋₁₈-aralkyl;
R³ denotes H, -C₁₋₆-alkyl, -OH, -O-C₁₋₆-alkyl, -O-C₇₋₁₈-aralkyl, -OOC-C₁₋₆-alkyl, -OOC-aryl or halogen;
or an acid addition salt thereof, starting from 1-amino-3-(phenyl)-propan-3-one of formula **II**, 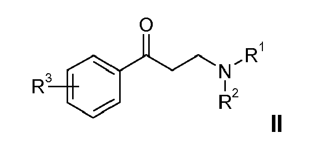 wherein R¹, R², and R³ are as hereinbefore defined, or an acid addition salt thereof, **characterised in that** the latter (II) is subjected to asymmetrical hydrogenation in the presence of a catalyst system consisting of rhodium and chiral 4-(dicyclohexylphosphino)-2-(diphenylphosphino-methyl)-N-methyl-aminocarbonyl-pyrrolidine, in the presence of less than one equivalent of a weak base selected from among the alkali metal hydrogen carbonates, alkali metal carbonates and in an inert protic diluent which contains water, and the following steps are carried out in order to isolate the product:
(i) dividing the reaction mixture obtained in the asymmetrical hydrogenation between water and an organic solvent,
(ii) adjusting the pH value of the aqueous phase in the acid range,
(iii) separating off the aqueous phase,
(iv) optionally repeating steps (i) to (iii)
(v) adjusting the pH value of the aqueous phase in the basic range;
(vi) dividing the reaction mixture between water and an organic solvent,
(vii) optionally repeating steps (v) to (vi)
(vii) separating off the organic phase formed and concentrating it.

2. Process according to claim 1 wherein
R¹ denotes H, methyl, ethyl, *iso*-propyl, *tert*-butyl or benzyl;
R² denotes H, methyl, ethyl, *iso*-propyl, *tert*-butyl or benzyl;
R³ denotes H, methyl, *iso*-propyl, *tert*-butyl, hydroxy, methoxy, propoxy, butoxy, benzyloxy, acetyloxy, benzoyloxy, F, Cl or Br.

3. Process for preparing *S*-3-hydroxy-3-phenyl-propylamines **I-S** according to one of claims 1 or 2, 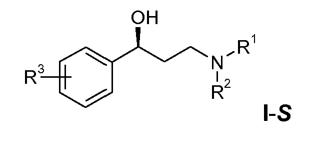 wherein the catalyst system consists of rhodium and (2*R*, 4*R*)-4-(dicyclo-hexylphosphino)-2-(diphenylphosphino-methyl)-N-methyl-aminocarbonyl-pyrrolidine.

4. Process for preparing *R*-3-hydroxy-3-phenyl-propylamines **I**-***R*** according to one of claims 1 or 2, 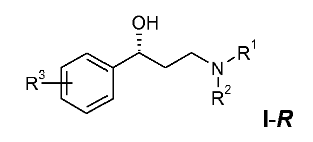 wherein the catalyst system consists of rhodium and (2*S*, 4*S*)-4-(dicyclo-hexylphosphino)-2-(diphenylphosphino-methyl)-N-methyl-aminocarbonyl-pyrrolidine.

5. Process according to one of claims 1 to 4, **characterised in that** the asymmetric hydrogenation is carried out in a temperature range of from 0°C to 100°C.

6. Process according to one of claims 1 to 5, **characterised in that** the asymmetric hydrogenation is carried out under a pressure of from 1 to 150 bar.

7. Process according to one of claims 1 to 6, wherein a compound of formula **II** or an acid addition salt thereof is used in a molar ratio of 500:1 to 100000:1 1 to the rhodium catalyst in the asymmetrical hydrogenation.

8. Process according to one of claims 1 to 7, wherein the rhodium catalyst for the asymmetric hydrogenation is used as a pre-prepared solution.

9. Process according to one of claims 1 to 7, wherein the rhodium catalyst for the asymmetric hydrogenation is prepared *in situ.*

10. Process according to one of claims 1-9, wherein the asymmetric hydrogenation is carried out within a reaction period of from 2 to 48 hours.

11. Process according to one of the preceding claims, wherein the product of the catalytic hydrogenation is subsequently converted into a salt.

12. Process for preparing R-atomoxetine, wherein first of all the compound **I-A** is prepared by the process according to one of claims 1 to 11, 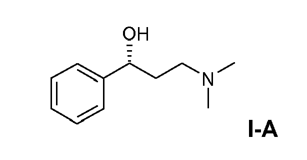 and is then reacted in subsequent reaction steps to form *R* -atomoxetine.

13. Process for preparing *R*-atomoxetine, wherein first of all the compound **I-B** is prepared by the process according to one of claims 1 to 11, 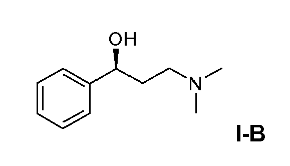 and is then reacted in subsequent reaction steps to form *R* -atomoxetine.

14. Process for preparing *R*-atomoxetine, wherein first of all the compound **I-C** is prepared by the process according to one of claims 1 to 11, 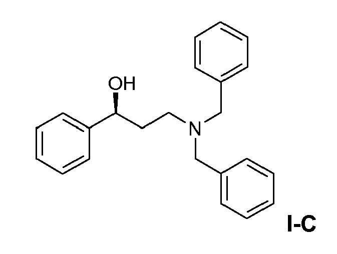 and is then reacted in subsequent reaction steps to form *R* -atomoxetine.

## Revendications

1. Procédé de production de 3-hydroxy-3-phényl-propylamines chirales, de formule I 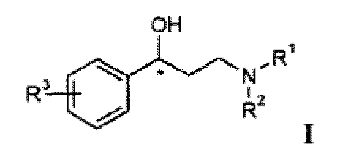 dans laquelle
R¹ est H, un groupe alkyle en C₁ à C₆ ou aralkyle en C₇ à C₁₈,
R² est H, un groupe alkyle en C₁ à C₆ ou aralkyle en C₇ à C₁₈ ;
R³ est H, un groupe alkyle en C₁ à C₆, -OH, -O-alkyle en C₁ à C₆, -O-aralkyle en C₇ à C₁₈, -OOC-alkyle en C₁ à C₆, -OOC-aryle ou un atome d'halogène ;
ou un sel d'addition acide de ceux-ci, à partir de 1-amino-3-(phényl)-propan-3-one de formule II, 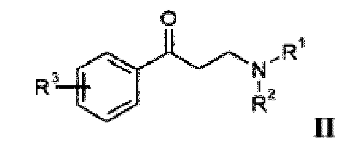 dans laquelle R¹, R² et R³ ont la signification indiquée ci-dessus, ou d'un sel d'addition acide de ceux-ci, **caractérisés en ce que** l'on soumet celui-ci (II) à une hydrogénation asymétrique en présence d'un système de catalyseur consistant en rhodium et 4-(dicyclohexylphosphino)-2-(diphényl- phosphino-méthyl)-N-méthyl-aminocarbonyl- pyrrolidine chiral en présence de moins d'un équivalent d'une base faible choisie dans le groupe des hydrogénocarbonates de métal alcalin, des carbonates de métal alcalin et dans un diluant protique inerte qui contient de l'eau, et pour l'isolement du produit, sont réalisées les étapes suivantes :
(i) répartition du mélange réactionnel obtenu lors de l'hydrogénation asymétrique entre l'eau et un solvant organique,
(ii) ajustement d'un pH de la phase aqueuse dans la plage acide,
(iii) séparation de la phase aqueuse,
(iv) éventuellement, répétition des étapes (i) à (iii)
(v) ajustement du pH de la phase aqueuse dans la plage basique ;
(vi) répartition du mélange réactionnel entre l'eau et un solvant organique,
(vii) éventuellement, répétition des étapes (v) à (vi)
(viii) séparation de la phase organique formée et concentration.

2. Procédé selon la revendication 1, dans lequel
R¹ est H, un groupe méthyle, éthyle, iso-propyle, tert-butyle ou benzyle ;
R² est H, un groupe méthyle, éthyle, iso-propyle, tert-butyle ou benzyle ;
R³ est H, un groupe méthyle, iso-propyle, tert-butyle, hydroxy, méthoxy, propoxy, butoxy, benzyloxy, acétyloxy, benzoyloxy, F, Cl ou Br.

3. Procédé pour la production de S-3-hydroxy-3-phényl-propylamines 1-S selon l'une des revendications 1 ou 2, 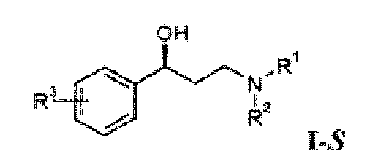 dans lequel le système de catalyseur consiste en rhodium et en (2R, 4R)-4-(dicyclohexylphosphino)-2-(diphénylphosphino-méthyl)-N-méthylaminocarbonylpyrrolidine.

4. Procédé pour la production de R-3-hydroxy-3-phényl-propylamines I-R selon l'une des revendications 1 ou 2, 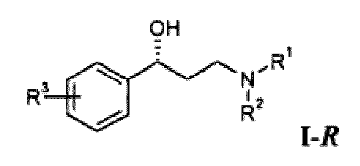 dans lequel le système de catalyseur consiste en rhodium et en (2S, 4S)-4-(dicyclohexylphosphino)-2-(diphénylphosphino-méthyl)-N-méthylaminocarbonylpyrrolidine.

5. Procédé selon l'une des revendications 1 à 4, **caractérisé en ce que** l'hydrogénation asymétrique est réalisée dans une plage de températures de 0°C à 100°C.

6. Procédé selon l'une des revendications 1 à 5, **caractérisé en ce que** l'hydrogénation asymétrique est réalisée à une pression de 1 à 150 bars.

7. Procédé selon l'une des revendications 1 à 6, dans lequel on utilise un composé de formule II ou un sel d'addition acide de celui-ci et le catalyseur de rhodium en un rapport molaire de 500:1 à 100000:1 lors de l'hydrogénation catalytique.

8. Procédé selon l'une des revendications 1 à 7, dans lequel le catalyseur de rhodium est utilisé pour l'hydrogénation asymétrique en tant que solution préalablement préparée.

9. Procédé selon l'une des revendications 1 à 7, dans lequel le catalyseur de rhodium pour l'hydrogénation asymétrique est produit in situ.

10. Procédé selon l'une des revendications 1 à 9, dans lequel l'hydrogénation asymétrique est réalisée pendant une durée réactionnelle de 2 à 48 heures.

11. Procédé selon l'une des revendications précédentes, dans lequel le produit de l'hydrogénation catalytique est ensuite converti en un sel.

12. Procédé pour la production de R-atomoxétine, dans lequel tout d'abord le composé I-A est produit conformément au procédé selon l'une des revendications 1 à 11 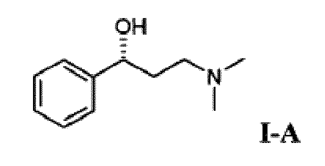 et ensuite, dans d'autres étapes réactionnelles, est converti en R-atomoxétine.

13. Procédé pour la production de R-atomoxétine, dans lequel tout d'abord le composé I-B est produit conformément au procédé selon l'une des revendications 1 à 11 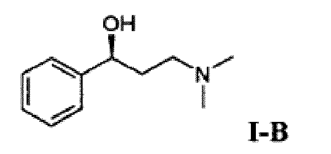 et ensuite, dans d'autres étapes réactionnelles, est converti en R-atomoxétine.

14. Procédé pour la production de R-atomoxétine, dans lequel tout d'abord le composé I-C est produit conformément au procédé selon l'une des revendications 1 à 11 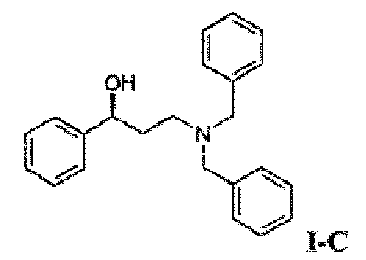 et ensuite, dans d'autres étapes réactionnelles, est converti en R-atomoxétine.
